# EUROPEAN PATENT APPLICATION

(11) **EP 3 858 317 A1**
(43) Date of publication of application: **04.08.2021**
(21) Application number: 19866837.8
(22) Date of filing: 04.09.2019
(51) Int. Cl.: A61F 13/514, A61F 13/15

(54) **SHEET MEMBER EXHIBITING UNEVENNESS, ABSORBENT ARTICLE EQUIPPED WITH SAME AND PRODUCTION METHOD THEREFOR**

(30) Priority: 27.09.2018 JP 2018182937
(71) Applicant: Daio Paper Corporation, Ehime 799-0492 (JP)
(72) Inventor: FURUKAWA, Masashi, Shikokuchuo-shi, Ehime 799-0431 (JP); OKADA, Yuki, Shikokuchuo-shi Ehime 799-0431 (JP)
(74) Representative: Williams Powell
(86) International application number: PCT/JP2019/034764
(87) International publication number: WO 2020/066511

(57) **Abstract**

To provide an absorbent article that improves an outward appearance of an absorbent article by a simple method.

A sheet member according to the invention includes a liquid impervious resin film 11, and a nonwoven fabric 12 attached to one surface thereof, in which a bonded portion in which the liquid impervious resin film 11 and the nonwoven fabric 12 are bonded through a micro-fibrous cellulose assembly 15 and a non-bonded portion continuously or intermittently provided between bonded portions are alternately repeatedly provided, and unevenness is formed on the nonwoven fabric 12 by the nonwoven fabric expanding in the non-bonded portion and the nonwoven fabric not expanding in the bonded portion.

## Description

### Technical Field

The present invention relates to a sheet member having unevenness, an absorbent article such as a disposable diaper such as an underpants type diaper or a tape-type diaper, or a sanitary napkin including the same, and a manufacturing method thereof.

### Background Art

When a consumer purchases a product, an outward appearance and hand feel of the product are important. For example, a disposable absorbent article such as a disposable diaper basically has a liquid impervious resin film on a back surface side of an absorber in order to prevent back leakage of liquid content of excrement. However, when the liquid impervious resin film is exposed on an outer surface of the product, the product does not look like a cloth. Therefore, it becomes common to place a nonwoven fabric on the outer surface of the product and make the outer surface of the product look like a cloth.

When the outer surface of the product is formed of the nonwoven fabric as described above, even though unevenness such as a wrinkle is rarely formed on the nonwoven fabric, unevenness may be intentionally formed for further improvement in flexibility and hand feel, improvement in appearance, or functional requirements. Naturally, there may be a demand for forming similar unevenness inside the product. In forming such unevenness, conventionally, embossing has been used to form the unevenness, or contraction wrinkles have been formed by an elastically stretchable member, etc.

However, even though these conventional methods are suitable for forming clear unevenness, the methods are not for forming gentle and natural textured unevenness.

### Citation List

### Patent Literature

Patent Literature 1: JP 5502742 B2
Patent Literature 2: JP 2018-108338 A

### Summary of Invention

### Technical Problem

Therefore, a main object of the invention is to provide a novel sheet member having gentle and natural textured unevenness and an absorbent article including the same.

### Solution to Problem

Sheet members and absorbent articles solving the above problems are as follows.

### <Invention recited in claim 1>

A sheet member including
a liquid impervious resin film, and
a nonwoven fabric attached to one surface thereof,
in which a bonded portion in which the liquid impervious resin film and the nonwoven fabric are bonded through a micro-fibrous cellulose assembly and a non-bonded portion continuously or intermittently provided between bonded portions are alternately repeatedly provided, and
unevenness is formed on the nonwoven fabric by the nonwoven fabric expanding in the non-bonded portion and the nonwoven fabric not expanding in the bonded portion.

### (Function and Effect)

The sheet member can be manufactured by a novel manufacturing method described later, and a bulge has a gentle and natural texture. Thus, for example, the sheet member has wrinkle-shaped unevenness as crepe.

Note that Patent Literature 1 describes an invention that utilizes micronized cellulose fibers in an absorbent article, which relates to a water-resistant and highly air-permeable composite sheet, and does not relate to an attachment structure of a sheet using a hot melt adhesive.

### <Invention recited in claim 2>

The sheet member according to claim 1,
in which the bonded portion with the micro-fibrous cellulose assembly is provided in a striped shape, and
the liquid impervious resin film and the nonwoven fabric are attached through a hot melt adhesive intermittently provided in a longitudinal direction of the bonded portion between the bonded portions.

### (Function and Effect)

In this way, unevenness is repeatedly formed in the longitudinal direction of the bonded portion between the bonded portions by the micro-fibrous cellulose assembly, and the liquid impervious resin film and a nonwoven fabric layer can be firmly bonded together.

### <Invention according to claim 3>

The sheet member recited in claim 1 or 2,
in which the micro-fibrous cellulose assembly in the bonded portion includes 0.3 to 5.0 g/m² of micro-fibrous cellulose.

### (Function and Effect)

When the attachment amount of the micro-fibrous cellulose assembly is within this range, the unevenness in the bonded portion and the periphery thereof has a gentle and natural texture.

### <Invention according to claim 4>

An absorbent article including
an absorber, and
the sheet member according to any one of claims 1 to 3 arranged on a back surface side of the absorber so that the liquid impervious resin film is on a side of the absorber,
in which at least a part of an outer surface of a product is formed by the nonwoven fabric.

### (Function and Effect)

The absorbent article has the wrinkle-shaped unevenness having a gentle and natural texture on the outer sheet included in the outer surface of the product.

### <Invention recited in claim 5>

A method of manufacturing a sheet member including a liquid impervious resin film and a nonwoven fabric attached to one surface thereof, the method including
applying a dispersion liquid of micro-fibrous cellulose at intervals to at least one of facing surfaces of the liquid impervious resin film and the nonwoven fabric, and
then bonding and drying the liquid impervious resin film and the nonwoven fabric, thereby alternately repeatedly providing a bonded portion in which the liquid impervious resin film and the nonwoven fabric are bonded through a micro-fibrous cellulose assembly and a non-bonded portion continuously or intermittently provided between bonded portions.

### (Function and Effect)

When a dispersion liquid of micro-fibrous cellulose is applied at intervals to at least one of facing surfaces of the liquid impervious resin film and the nonwoven fabric, and then the liquid impervious resin film and the nonwoven fabric are bonded and dried, it is possible to alternately repeatedly provide a bonded portion in which the liquid impervious resin film and the nonwoven fabric are bonded through a micro-fibrous cellulose assembly and a non-bonded portion continuously or intermittently provided between bonded portions. Here, when the micro-fibrous cellulose dispersion liquid attached to the liquid impervious resin film and the nonwoven fabric is dried to form the micro-fibrous cellulose assembly, the volume of the micro-fibrous cellulose assembly gradually decreases. For this reason, the micro-fibrous cellulose assembly not only bonds the liquid impervious resin film and the nonwoven fabric as an adhesive, but also contracts together with the liquid impervious resin film and the nonwoven fabric. In this instance, in the bonded portion, the liquid impervious resin film has wrinkles. However, the nonwoven fabric can contract to some extent and may not freely expand by being bonded to the liquid impervious resin film through the micro-fibrous cellulose assembly, and thus do not have wrinkles. On the other hand, the non-bonded portion is continuously or intermittently formed between the bonded portions, and the nonwoven fabric in the non-bonded portion contracts together with the nonwoven fabric in the bonded portion. As a result, the nonwoven fabric expands in the non-bonded portion, and the nonwoven fabric does not expand in the bonded portion, so that the wrinkle-shaped unevenness having a gentle and natural texture is formed on the nonwoven fabric.

Furthermore, this manufacturing method only adds a process of attaching the dispersion liquid of micro-fibrous cellulose to at least one of the liquid impervious resin film and the nonwoven fabric and drying the dispersion liquid in order to form the unevenness. Therefore, the unevenness can be formed by a significantly simple method.

### <Invention recited in claim 6>

A method of manufacturing an absorbent article including an absorber, and the liquid impervious resin film and a nonwoven fabric in this order on a back surface side of the absorber, at least a part of an outer surface of a product being formed by the nonwoven fabric, the method including
arranging a sheet member formed by the method recited in claim 5 so that the liquid impervious resin film is on a side of the absorber.

### (Function and Effect)

According to this manufacturing method, it is possible to manufacture an absorbent article having wrinkle-shaped unevenness having a gentle and natural texture on the nonwoven fabric included in the outer surface of the product by a simple method.

### Advantage Effects of Invention

The invention obtains a novel sheet member having gentle and natural textured unevenness and an absorbent article including the same.

### Brief Description of Drawings

Fig. 1 is a plan view illustrating an inner surface of a tape-type disposable diaper in a state where a diaper is spread.
Fig. 2 is a plan view illustrating an outer surface of the tape-type disposable diaper in a state where a diaper is spread.
Fig. 3 is a cross-sectional view taken along line 6-6 of Fig. 1.
Fig. 4 is a cross-sectional view taken along line 7-7 of Fig. 1.
Fig. 5 is a cross-sectional view taken along line 8-8 of Fig. 1.
Fig. 6 is a cross-sectional view taken along line 9-9 of Fig. 1.
Fig. 7 is a cross-sectional view taken along line 5-5 of Fig. 1.
Fig. 8 is an explanatory view illustrating arrangement of a micro-fibrous cellulose assembly.
Fig. 9 is an explanatory view illustrating arrangement of an adhesive and the micro-fibrous cellulose assembly.
Fig. 10 is a cross-sectional view taken along line X-X of Fig. 9.
Fig. 11 is an explanatory view illustrating arrangement of the adhesive and the micro-fibrous cellulose assembly.
Fig. 12 is a cross-sectional view taken along line Y-Y of Fig. 11.
Fig. 13 is an explanatory view illustrating arrangement of the adhesive and the micro-fibrous cellulose assembly.
Fig. 14 is an explanatory view illustrating arrangement of the adhesive and the micro-fibrous cellulose assembly.
Fig. 15 is a cross-sectional view taken along line Z-Z of Fig. 14.
Fig. 16 is an explanatory view illustrating arrangement of the adhesive and the micro-fibrous cellulose assembly.
Fig. 17 is an explanatory view illustrating arrangement of the adhesive and the micro-fibrous cellulose assembly.
Fig. 18 is a diagram illustrating formation of unevenness.
Fig. 19 is a diagram illustrating formation of unevenness.
Fig. 20 is an explanatory view of an application example of a hot melt adhesive.

### Description of Embodiments

Hereinafter, a tape-type disposable diaper will be described as an example for carrying out the invention. Figs. 1 to 7 illustrate examples of a tape-type disposable diaper, in which reference character X indicates a maximum width of the diaper excluding a connecting tape, reference character L indicates a maximum length of the diaper, and respective constituent members in cross-sectional views are bonded by an adhesive as bonding means for bonding. The application of adhesive is performed by solid, bead, curtain, summit, or spiral coating of a hot melt adhesive, or pattern coating (transfer of the hot melt adhesive in a letterpress method), or application of an elastic member to an outer peripheral surface such as comb gun or sure wrap application instead of or together with the above methods in a fixed part of the elastic member. Examples of a hot melt adhesive 81 include EVA-based, pressure sensitive adhesion rubber-based (elastomer-based), polyolefin-based, and polyester/polyamide-based adhesives, and can be used without any particular limitation (however, the adhesive does not include a micro-fibrous cellulose assembly 15). As bonding means that bonds respective components, it is possible to use means by material welding such as heat sealing or ultrasonic sealing. Specifically, in Figs. 3 and 4, in a region in which a liquid impervious resin film 11 and an outer nonwoven fabric 12 are bonded to each other (hereinafter referred to as a "bonded region 82"), a bonding may be performed by the hot melt adhesive 81 and the micro-fibrous cellulose assembly 15. In this case, a basis weight of the hot melt adhesive 81 applied to a linear adhesive part of the hot melt adhesive 81 can be set to 1 to 20 g/m². Within this range, the liquid impervious resin film 11 and the outer nonwoven fabric 12 are firmly attached to each other at an application portion of the hot melt adhesive 81 in the bonded region 82.

In addition, a dotted pattern portion indicated by a square in the cross-sectional views illustrates a pressure sensitive adhesive as bonding means for bonding the respective constituent members located on the front surface side and the back surface side thereof, and is formed by application to the respective constituent members by a dispersion liquid of micro-fibrous cellulose described in detail below.

This tape-type disposable diaper includes an absorber 56, a liquid pervious top sheet 30 that covers a front surface side of the absorber 56, a liquid impervious resin film 11 that convers an outer side of the absorber 56, and an outer nonwoven fabric 12 that covers an outer side of the liquid impervious resin film and forms an outer surface of the product. Reference character F indicates a ventral side part located on a front side of a center in a front-back direction, and reference character B indicates a dorsal side part located on a back side of the center in the front-back direction.

Materials and characteristic parts of each portion will be described below in order.

### (Absorber)

The absorber 56 is a part that absorbs and retains body fluids such as excreted liquid and blood, and can be formed of an assembly of fibers. As this fiber assembly, in addition to an assembly obtained by accumulating short fibers such as fluff pulp or synthetic fibers, it is possible to use a filament assembly obtained by opening a tow (fiber bundle) of synthetic fibers such as cellulose acetate as necessary. A fiber basis weight can be set to, for example, about 100 to 300 g/m² in the case of accumulating fluff pulp or short fibers, and can be set to, for example, about 30 to 120 g/m² in the case of the filament assembly. The fineness of the synthetic fiber is, for example, 1 to 16 dtex, preferably 1 to 10 dtex, and more preferably 1 to 5 dtex. In the case of the filament assembly, the filament may be non-crimped fiber, and is preferably crimped fiber. The crimp degree of the crimped fibers can be, for example, about 5 to 75, preferably 10 to 50, and more preferably about 15 to 50 per 2.54 cm. In addition, crimped fibers that are uniformly crimped can be used.

### (Super Absorbent Polymer Particles)

The absorber 56 may contain super absorbent polymer particles in a part or all thereof. Super absorbent polymer particles include "powder" in addition to "particles". As the super absorbent polymer particles 54, those used for this type of absorbent articles can be used on an as-is basis. A particle size of the super absorbent polymer particles is not particularly limited. For example, it is desirable to use those in which when sieving (shaking for 5 minutes) using a standard sieve (JIS Z8801-1:2006) of 500 µm and sieving (shaking for 5 minutes) using a standard sieve (JIS Z8801-1:2006) of 180 µm for particles falling under the former sieve are performed, a ratio of particles remaining on the standard sieve of 500 µm is 30% by weight or less, and a ratio of particles remaining on the standard sieve of 180 µm is 60% by weight or more.

The material of the super absorbent polymer particles can be used without particular limitation, but the material having the water absorption capacity of 30 g/g or more is suitable. As the super absorbent polymer particles, starch-based, cellulose-based, and synthetic polymer-based, and starch-polyacrylate (salt) graft copolymers, saponified starch-acrylonitrile copolymers, sodium carboxymethyl cellulose crosslinked products, acrylic acid (salt) polymers, and the like can be used. As the shape of the super absorbent polymer particles, the shapes of particulate materials which are usually used are suitable, but other shapes can also be used.

The super absorbent polymer particles having a water absorption speed of 70 seconds or less, particularly 40 seconds or less, are suitably used. If the water absorption speed is too slow, back-flow, in which the liquid fed into the absorber 56 returns to the outside of the absorber 56, is likely to occur.

In addition, the super absorbent polymer particles having the gel strength of 1,000 Pa or more are preferably used. Thereby, even when the absorber 56 is bulky, it is possible to effectively suppress stickiness after liquid absorption.

The basis weight of the super absorbent polymer particles can be appropriately determined according to the absorption amount required for the use of the absorber 56. Therefore, although it cannot be said unconditionally, the basis weight can be 50 to 350 g/m². When the basis weight of the polymer is less than 50 g/m², it is difficult to secure the absorption amount. When 350 g/m² is exceeded, not only the effect is saturated, but also the excessive amount of super absorbent polymer particles gives a gritty and uncomfortable feeling.

### (Wrapping Sheet)

To prevent super absorbent polymer particles from leaking out, or to improve a shape maintaining property of the absorber 56, the absorber 56 can be incorporated as an absorbent element 50 wrapped with the wrapping sheet 58. As the wrapping sheet 58, tissue paper, particularly crepe paper, a nonwoven fabric, a polyethylene laminated nonwoven fabric, a sheet with small holes, and the like can be used. However, it is desirable that the wrapping sheet be a sheet through which the super absorbent polymer particles do not pass. When a nonwoven fabric is used instead of crepe paper, a hydrophilic SMMS (spun bond/melt blown/melt blown/spun bond) nonwoven fabric is particularly suitable, and polypropylene, polyethylene/polypropylene, etc. can be used as a material. It is desirable that the basis weight of the fiber is 5 to 40 g/m², desirably 10 to 30 g/m².

As illustrated in Fig. 3, the entire absorber 56 may be wrapped with one wrapping sheet 58. Alternatively, the entire absorber 56 may be wrapped with a plurality of sheets such as two upper and lower sheets, and the wrapping sheet 58 may be omitted.

### (Top Sheet)

The top sheet 30 has a liquid pervious property. For example, it is possible to use a perforated or imperforate nonwoven fabric, a porous plastic sheet, etc. In addition, a raw material fiber of the nonwoven fabric is not particularly limited. Examples of the constituent fibers can include synthetic fibers such as polyolefin-based synthetic fibers such as polyethylene and polypropylene, polyester-based synthetic fibers, and polyamide-based synthetic fibers, regenerated fibers such as rayon and cupra, natural fibers such as cotton, mixed fibers and conjugate fibers in which two or more of these are used, and the like. Further, the nonwoven fabric may be manufactured by any processing. Examples of processing methods can include known methods such as a spun lace method, a spun bond method, a thermal bond method, a melt blown method, a needle punch method, an air through method, and a point bond method. For example, the spun lace method is preferable when flexibility and drapeability are required, and the thermal bond method is preferable when bulkiness and softness are required.

The top sheet 30 extends from a front end to a back end of the product in the front-back direction, and extends laterally from the absorber 56 in the width direction WD. However, for example, when starting points of rising gathers 60 described later are located on center sides of side edges of the absorber 56 in the width direction, appropriate deformation such as making the width of the top sheet 30 shorter than the maximum width of the absorber 56 can be made as necessary.

### (Intermediate Sheet)

To rapidly transfer the liquid permeating the top sheet 30 to the absorber, it is possible to provide an intermediate sheet (also referred to as "second sheet") 40 having a higher liquid permeation rate than that of the top sheet 30. The intermediate sheet 40 is intended to rapidly transfer the liquid to the absorber to enhance the absorption performance of the absorber and prevent a "returning" phenomenon of the absorbed liquid from the absorber. The intermediate sheet 40 can be omitted.

Examples of the intermediate sheet 40 can include the same material as that of the top sheet 30, a spun lace nonwoven fabric, a spun bond nonwoven fabric, an SMS nonwoven fabric, a pulp nonwoven fabric, a mixed sheet of pulp and rayon, a point bond nonwoven fabric, or crepe paper. In particular, an air through nonwoven fabric is bulky, and thus is preferable. It is preferable to use a composite fiber having a core-sheath structure for the air through nonwoven fabric. In this case, a resin used for the core may be polypropylene (PP), and is preferably polyester (PET) having high rigidity. A basis weight is preferably 17 to 80 g/m², more preferably 25 to 60 g/m². A fineness of a raw material fiber of the nonwoven fabric is preferably 2.0 to 10 dtex. In order to make the nonwoven fabric bulky, it is preferable to use eccentric fibers having no core in the center, hollow fibers, or eccentric and hollow fibers as all of raw material fibers or a part of mixed fibers .

Although the intermediate sheet 40 in the illustrated example is shorter than the width of the absorber 56 and disposed at the center portion, it may be provided over the maximum width. Further, the intermediate sheet 40 may be provided over the maximum length of the diaper, and may be provided only at an intermediate part including an excretion position as in the illustrated example.

### (Liquid Impervious Resin Film)

The liquid impervious resin film 11 is not particularly limited as long as the liquid impervious resin film 11 has moisture permeability. For example, a microporous sheet can be suitably used which is obtained by kneading a polyolefin-based resin such as polyethylene or polypropylene and an inorganic filler, molding the kneaded materials into a sheet and monoaxially or biaxially stretching the sheet. In particular, naturally, the liquid impervious resin film 11 may be one having moisture permeability in the thickness direction. However, the liquid impervious resin film 11 does not include a material containing a nonwoven fabric as a base material and having enhanced waterproofness.

The liquid impervious resin film 11 made of a resin film having moisture permeability may be subjected to intermittent decorative printing such as product logo, character picture, photograph, etc. that is placed on either or both front and back sides of the product, in addition to continuous decorative printing including a plurality of constituent units such as characters that are regularly repeated in the front-back direction LD and width direction WD (size, brand name, maker name, design name, etc.) and patterns. When such decorative printing is performed, it is desirable that the liquid impervious resin film 11 has a small elongation.

It is desirable that the liquid impervious resin film 11 extends in the front-back direction LD and the width direction WD in the same or wider range as or than that of the absorber 56. However, when other water-impervious means are present, etc., as necessary, an end portion of the absorber 56 may not be covered in the front-back direction LD and the width direction WD. On the center side of the inner surface of the liquid impervious resin film 11 in the width direction, an indicator whose color changes or disappears by the liquid content of the excrement can be provided to have a length that is 1/3 to 3/4 of the maximum length L in the front-back direction. As the indicator, a known one can be used without particular limitation. For example, the indicator can be configured by a sheet-shaped member containing a coloring agent having a color reaction upon contact with water in excrement and/or a coloring agent having a color reaction by detecting pH in water, or ink or adhesive containing a drug having a reaction in which coloring disappears due to a reaction with liquid content of the excrement, a reaction in which a coloring agent dissolves (disperses) in the excreted liquid to spread or disappear, or other visual changes, or a chemical agent having a visual change upon contact with water or liquid in excrement (indicator reaction means). As the coloring agent having a color reaction upon contact with water in excrement, it is possible to use a coloring agent containing a water-soluble or water-decomposable dye or leuco dye and a developer such as a phenolic compound, an acidic substance or an electron-accepting substance causing the leuco dye to develop a color. The color change due to the indicator affects the appearance of the absorbent article.

### (Outer Nonwoven Fabric)

The outer nonwoven fabric 12 covers the entire back surface side of the liquid impervious resin film 11 and makes the outer surface of the product look like a cloth. The outer nonwoven fabric 12 is not particularly limited. As a raw material fiber, for example, in addition to synthetic fibers such as polyolefin-based fiber such as polyethylene or polypropylene, polyester-based fiber, and polyamide-based fiber, regenerated fibers such as rayon and cupra, and natural fibers such as cotton can be used. As a processing method, a spun lace method, a spun bond method, a thermal bond method, an air through method, a needle punch method, and the like can be used. However, a long-fiber nonwoven fabric such as a spunbonded nonwoven fabric, an SMS nonwoven fabric, and an SMMS nonwoven fabric is preferable in that good texture and strength can be compatible. In addition to using a single piece of nonwoven fabric, it is also possible to use multiple nonwoven fabrics in layers. In the latter case, it is preferable that the nonwoven fabrics are attached to each other with a hot melt adhesive or the like. When a nonwoven fabric is used, it is desirable that the basis weight of the fiber is 10 to 50 g/m², particularly 15 to 30 g/m².

### (Rising Gather)

In order to prevent excrement that moves laterally on the top sheet 30 and prevent so-called side leakage, the rising gathers 60 standing up to the skin side of the wearer are preferably provided on both sides of the surface in the width direction WD. Naturally, the rising gathers 60 can be omitted.

When the rising gathers 60 are adopted, a structure thereof is not particularly limited, and any known structure can be adopted. Each of the rising gathers 60 of the illustrated example includes a gather sheet 62 that is substantially continuous in the width direction WD and an elongated gather elastic member 63 fixed to the gather sheet 62 in a stretched state along the front-back direction LD. As this gather sheet 62, a water repellent nonwoven fabric can be used, and rubber thread or the like can be used as the gather elastic member 63. As illustrated in Figs. 1 and 2, a plurality of the elastic members may be provided on each side, or only one elastic member may be provided on each side.

The inner surface of the gather sheet 62 has a joint start point in the width direction WD on the side portion of the top sheet 30, and a part from the joint start point to an outer side in the width direction is bonded to an inner surface of each side flap portion SF, that is, a side portion of the liquid impervious resin film 11 and a side portion of the outer nonwoven fabric 12 located on the outer side thereof in the width direction in the illustrated example by a hot melt adhesive, etc.

In the periphery of the leg, the inside in the width direction from the joint start point of each rising gather 60 is fixed on the top sheet 30 at both ends of the product in the front-back direction. However, the portion therebetween is a non-fixed free portion, and this free point is erected by contraction force of the elastic member 63 and comes into close contact with a body surface.

### (End Flap Portion and Side Flap Portion)

The tape-type disposable diaper of the illustrated example includes a pair of end flap portions EF that extends to the front side and the back side of the absorber 56, respectively, and does not have the absorber 56, and a pair of side flap portion SF that extends laterally beyond both side edges of the absorber 56 and does not have the absorber 56.

### (Flat Gather)

Side elastic members 64 made of an elongated elastic member such as rubber thread, etc. are fixed to the respective side flap portions SF in a state of being extended along the front-back direction LD. In this way, around-leg parts of the respective side flap portions SF are configured as flat gathers. The around-leg elastic members 64 may be provided between the gather sheet 62 and the liquid impervious resin film 11 on the outer side in the width direction near the joint start point in the bonded portion of the gather sheet 62 as in the illustrated example, or may be provided between the liquid impervious resin film 11 and the outer nonwoven fabric 12 in the side flap portions SF. As in the illustrated example, a plurality of around-leg elastic members 64 may be provided on each side, or only one elastic member 64 may be provided on each side.

### (Connecting Tape)

Connecting tapes 13 detachably connected to the outer surface of the ventral side part F are provided in the side flap portions SF in the dorsal side part B, respectively. When the diaper 10 is worn, the connecting tapes 13 are turned from both sides of the waist to the outer surface of the ventral side part F, and connecting portions 13A of the connecting tapes 13 are connected to proper positions on the outer surface of the ventral side part F.

A structure of the connecting tapes 13 is not particularly limited. However, in the illustrated example, each connecting tape includes a sheet base material forming a tape attaching portion 13C fixed to the side flap portion SF and a tape main unit section 13B projecting from the tape attaching portion 13C, and a connecting portion 13A with respect to the ventral side, which is provided in the intermediate portion in the width direction of the tape main unit section 13B in the seat base material. A tip end side from the connecting portion 13A is a tab part.

As the connecting portion 13A, a hook material (hook member) of a mechanical fastener (hook and loop fastener) may be provided, or an adhesive layer may be provided. The hook material has a plurality of engagement projections on a connecting surface thereof, and the engagement projection has a (A) check mark shape, a (B) J shape, a (C) mushroom shape, a (D) T shape, and a (E) double J shape (a shape bonded back to back of a J shape), but may have any shape.

Further, as the sheet base material forming from the tape attaching portion 13C to the tape main unit section 13B, it is possible to use a nonwoven fabric, a plastic film, poly-laminated nonwoven fabric, paper and a composite material thereof. However, it is preferable to use a spun bond nonwoven fabric, an air through nonwoven fabric, or a spun lace nonwoven fabric having a fineness of 1.0 to 3.5 dtex, a basis weight of 20 to 100 g/m², and a thickness of 1 mm or less.

### (Target Sheet)

It is preferable to provide a target sheet 20 having a target for facilitating connection at the connection portion of each connecting tape 13 in the ventral side part F. In a case where the connecting portion 13A is the hook member, the target sheet 20 can be used having a large number of loops made of threads to which engagement projections of the hook member are tangled provided on a surface of the sheet base material made of a plastic film or a nonwoven fabric. Further, in the case of a pressure sensitive adhesion material layer, it is possible to use a sheet base material made of a plastic film having a smooth surface with high adhesiveness and subjected to a release treatment. In addition, in a case where the connection portions of the connecting tapes 13 in the ventral side part F are made of the nonwoven fabric, for example, in the case of having the outer nonwoven fabric 12 as in the illustrated embodiment, the target sheet 20 may be omitted, and the hook material may be entangled and connected to the fibers of the outer nonwoven fabric 12. In this case, the target sheet 20 as a mark may be provided between the outer nonwoven fabric 12 and the liquid impervious resin film 11.

### (Micro-fibrous Cellulose)

Micro-fibrous cellulose refers to fine cellulose fibers obtained by defibrating pulp fibers, and generally refers to cellulose fibers containing cellulose fine fibers having an average fiber width of nano size (1 nm or more and 1000 nm or less). However, those having an average fiber width (median diameter) of 100 nm or less (generally referred to as cellulose nanofibril (CNF)) are preferable, and those having an average fiber width of 10 to 60 nm are particularly preferable. In addition, cellulose fibers include innumerable β-glucose units linked mainly by β-1,4 glycoside bonds in a chain. β-glucose has a -H group, a - OH group, etc.

Micro-fibrous cellulose has an effect of absorbing moisture (water molecules, etc.) and reducing odor. Micro-fibrous cellulose generally has a fiber width of 4 nm or more and 1000 nm or less, a fiber length of 5 µm or more, a high aspect ratio (5 or more for low, 1250 or more for high), and a large specific surface area, and is excellent in physical adsorption. A reason why the micro-fibrous cellulose has absorbability and odor reducing property is not clear. However, it is considered that one of the reasons is probably a moisture component and an odorous component are physically adsorbed on the surface of the micro-fibrous cellulose and retained so that a degree of freedom is lost. Further, since the micro-fibrous cellulose has a molecular structure having a large number of -OH groups, micro-fibrous cellulose and moisture (water molecules, etc.) have a high affinity.

The dispersion liquid of micro-fibrous cellulose is obtained by dispersing micro-fibrous cellulose in a solvent. The concentration (mass/volume) of the dispersion liquid of micro-fibrous cellulose is preferably 0.1 to 10%, more preferably 1.0 to 5.0%, and particularly preferably 1.5 to 3.0%. The solvent in which micro-fibrous cellulose is dispersed is not particularly limited, and it is possible to use water, lower alcohol such as ethanol, or a volatile organic solvent such as acetone.

The B-type viscosity (60 rpm, 20°C) of the dispersion liquid of micro-fibrous cellulose is, for example, 700 cps or less, preferably 200 cps or less, and more preferably 50 cps or less. By keeping the B-type viscosity of micro-fibrous cellulose dispersion liquid low in this way, the micro-fibrous cellulose assembly 15 is evenly applied to the sheet surface, and the surface properties of the sheet are improved uniformly.

Since the micro-fibrous cellulose assembly 15 has adhesiveness (that is, without an adhesive), the micro-fibrous cellulose assembly 15 can have an adhesive function even though the adhesive force varies depending on the object to be attached.

A method for measuring the average fiber width of micro-fibrous cellulose will be described.

First, 100 ml of an aqueous dispersion liquid of micro-fibrous cellulose having a solid content concentration of 0.01 to 0.1% by mass is filtered through a Teflon (registered trademark) membrane filter, and the solvent substitutions are performed once with 100 ml of ethanol and three times with 20 ml of t-butanol.

Next, freezing drying and coating with osmium are performed to obtain a sample. This sample is observed with an electron microscope SEM image at a magnification of 5,000 times, 10,000 times or 30,000 times (magnification of 30,000 times in this example) depending on the width of the constituent fibers. Specifically, two diagonal lines are drawn on the observed image, and three straight lines passing through an intersection of the diagonal lines are arbitrarily drawn. Further, a total of 100 fiber rods intersecting these three straight lines are visually measured. Then, a median diameter of the measured values is taken as the average fiber width. Note that an average fiber diameter is not limited to the median diameter of the measured values. For example, a number-average diameter or a mode diameter (most frequent diameter) may be used as the average fiber diameter.

Examples of pulp fibers that can be used to produce micro-fibrous cellulose include chemical pulp such as hardwood pulp (LBKP) or softwood pulp (NBKP), mechanical pulp such as bleached thermomechanical pulp (BTMP), stone ground pulp (SGP), pressure stone ground pulp (PGW), refiner ground pulp (RGP), chemi-ground pulp (CGP), thermo-ground pulp (TGP), ground pulp (GP), thermo-mechanical pulp (TMP), chemi-thermo-mechanical pulp (CTMP), or refiner mechanical pulp (RMP), waste paper pulp manufactured from tea waste paper, craft envelope blind paper, magazine waste paper, newspaper waste paper, flyer waste paper, office waste paper, cardboard waste paper, upper white paper, Kent waste paper, imitation waste paper, land certificate waste paper, or coarse paper waste paper, and deinked pulp (DIP) obtained by deinking waste paper pulp. These pulp fibers may be used alone or in combination of two or more types as long as the effects of the invention are not impaired. Furthermore, the pulp fibers subjected to chemically treatment such as carboxymethylation may be used.

Examples of the method for producing micro-fibrous cellulose include mechanical methods such as a highpressure homogenizer method, a microfluidizer method, a grinder grinding method, a bead mill freeze-grinding method, and an ultrasonic defibration method, and the method is not limited to these methods. Further, formation of fine fibers is promoted by the combined use of TEMPO oxidation treatment, phosphoric acid esterification treatment, acid treatment, etc.

When a consumer purchases a product, an outward appearance and hand feel of the product are important. For example, in general, a disposable absorbent article such as a disposable diaper has a liquid impervious resin film on a back surface side of an absorber in order to prevent back leakage of liquid content of excrement, and the nonwoven fabric is arranged on the outer surface thereof, so that the outer surface of the product has a cloth-like appearance. Even though unevenness such as a wrinkle is rarely formed on the nonwoven fabric, unevenness may be intentionally formed for further improvement in flexibility and hand feel, improvement in appearance, or functional requirements. The inventors payed attention to this point, and have found during earnest research that, when a predetermined amount of the micro-fibrous cellulose assembly 15 is attached to the liquid impervious resin film 11, unevenness 91 is formed in the attached part of the micro-fibrous cellulose assembly 15 and a periphery thereof.

Based on this fact, the invention relates to forming the unevenness 91 having a shape of a wrinkle by attaching the micro-fibrous cellulose assembly 15 to the liquid impervious resin film 11 and the outer nonwoven fabric 12 which are constituent members of the absorbent article.

In a conventional absorbent article, the liquid impervious resin film 11 and the outer nonwoven fabric 12 are bonded by an adhesive such as a hot melt adhesive as an example. In the embodiment of the invention, the micro-fibrous cellulose assembly 15 is attached and bonded to at least one of the liquid impervious resin film 11 or the nonwoven fabric 12.

The micro-fibrous cellulose assembly 15 is sufficiently attached to the liquid impervious resin film 11, and attached to the nonwoven fabric 12 to some extent not as much as the micro-fibrous cellulose assembly 15 is attached to the liquid impervious resin film 11.

Comparing the liquid impervious resin film 11 and the nonwoven fabric 12, the liquid impervious resin film 11 is produced by kneading a polyolefin-based resin and an inorganic filler and molding the kneaded mixture, and has relatively small voids. On the other hand, the nonwoven fabric 12 is formed by the spun lace method or another method, and has relatively a lot of voids.

The unevenness formed on the bonded portion of the micro-fibrous cellulose assembly 15 and the periphery thereof is easily formed on a sheet having less voids, that is, a sheet on which a force is more easily transmitted.

As a result, the wrinkle-shaped unevenness 91 is clearly formed when the micro-fibrous cellulose assembly 15 is attached to the liquid impervious resin film 11. In the bonded portion, the liquid impervious resin film has wrinkles. However, the nonwoven fabric can contract to some extent and may not freely expand by being bonded to the liquid impervious resin film through the micro-fibrous cellulose assembly, and thus do not have wrinkles.

An example of a method of forming unevenness by attaching the micro-fibrous cellulose assembly 15 will be described. First, the micro-fibrous cellulose assembly 15 is attached to the liquid impervious resin film 11. The hot melt adhesive 81 is applied to the liquid impervious resin film 11 to which the micro-fibrous cellulose assembly 15 is attached. The hot melt adhesive 81 may be applied over the bonded portion of the micro-fibrous cellulose assembly 15, or may be applied except for the bonded portion. The nonwoven fabric 12 is bonded to the liquid impervious resin film 11 to which the hot melt adhesive 81 is applied using, for example, a roller. In this way, the unevenness 91 is formed on the bonded portion of the micro-fibrous cellulose assembly 15 in the liquid impervious resin film 11 and the periphery thereof. Then, the unevenness 91 is formed in the periphery of the bonded portion of the micro-fibrous cellulose assembly 15 in the nonwoven fabric 12.

In addition, since the micro-fibrous cellulose assembly 15 has adhesiveness, the sheet can be bonded without using the hot melt adhesive 81. An example of a method of forming unevenness by attaching the micro-fibrous cellulose assembly 15 in the case of not using will be described.
The micro-fibrous cellulose assembly 15 is attached to the liquid impervious resin film 11. The nonwoven fabric 12 is bonded to the liquid impervious resin film 11 to which the micro-fibrous cellulose assembly 15 is attached using, for example, a roller.

The bonded portion to which the micro-fibrous cellulose assembly 15 is attached in the liquid impervious resin film 11 unavoidably hard. Then, the unevenness 91 is formed at the bonded portion and the periphery thereof. For example, when the bonded portion is linear, the unevenness is formed along the linear bonded portion. A mechanism by which the unevenness is formed is as follows. When the micro-fibrous cellulose dispersion liquid attached to the liquid impervious resin film 11 is dried, the micro-fibrous cellulose dispersion liquid contracts as the micro-fibrous cellulose assembly 15. In this instance, the liquid impervious resin film 11 that is bonded contracts. Due to this contraction, the unevenness 91 is formed on the bonded portion of the liquid impervious resin film 11 and the periphery thereof.

To form the unevenness 91 by the bonded portion of the micro-fibrous cellulose assembly 15, the amount of attachment to the target surface (bonded region 82) is preferably set to 0.3 to 5.0 g/m², and suitably set to 0.5 to 1.5 g/m². The micro-fibrous cellulose assembly 15 has air permeability, and has absorbability and odor reducing property. Further, when the attachment amount of the micro-fibrous cellulose assembly 15 is set to 0.5 to 1.5 g/m², the effects of air permeability, absorbability, and odor reducing property are outstanding. When the attachment amount is excessively small, the bonded portion does not have sufficient hardness, and the unevenness 91 is difficult to form. When the attachment amount is excessively large, the bonded portion becomes unnecessarily hard. However, when this range is exceeded, the unevenness 91 is formed.

Fig. 18 illustrates an example of the sheet member formed by the above method. This figure is a plan view of a part of the sheet member including the liquid impervious resin film 11 and the nonwoven fabric 12 as seen from the nonwoven fabric 12 side. In the bonded region of the liquid impervious resin film 11 and the nonwoven fabric 12, linear parts of the bonded portion of the micro-fibrous cellulose assembly 15 are arranged in an up-down direction in plan view and provided at intervals in a left-right direction. A plurality of pieces of unevenness 91 is formed between the linear parts. A shape of the unevenness 91 does not have regularity as when embossing is applied. Therefore, the unevenness 91 gives a gentle and natural texture.

The micro-fibrous cellulose assembly 15 can be manufactured by a known method in which the micro-fibrous cellulose assembly 15 is dispersed in a solvent to form a micro-fibrous cellulose dispersion liquid, and the micro-fibrous cellulose dispersion liquid is applied to a target sheet such as the liquid impervious resin film 11 and dried, thereby attaching and forming the micro-fibrous cellulose assembly 15 on the target sheet. As a drying method, it is possible to adopt known methods such as natural drying and drying by blowing hot air (hot air drying). According to these methods, unevenness is formed at the bonded portion of the micro-fibrous cellulose assembly 15 formed on the target sheet and the periphery thereof. Further, hot air drying for rapid drying is suitable considering the production efficiency of the product. Furthermore, when the target sheet is dried at the glass transition temperature or higher, the unevenness 91 is formed not only on the attachment portion (bonded portion) of the micro-fibrous cellulose assembly 15 and the periphery thereof on the target sheet but also on the target sheet. On the other hand, drying at a temperature lower than the glass transition temperature of the target sheet is preferable since the unevenness 91 is formed on the bonded portion and the periphery thereof.

Note that when the micro-fibrous cellulose dispersion liquid is attached to a fiber sheet such as paper or nonwoven fabric by the manufacturing method for attaching the micro-fibrous cellulose dispersion liquid in this way, even though the micro-fibrous cellulose dispersion liquid partially penetrates into the sheet, since the micro-fibrous cellulose assembly 15 concentrates on the surface, the micro-fibrous cellulose assembly 15 can be attached and formed on the sheet. Characteristically, the nonwoven fabric 12 can contract to some extent and may not freely expand by being bonded to the liquid impervious resin film 11 through the micro-fibrous cellulose assembly 15, and thus a bonded portion of the nonwoven fabric 12 does not have wrinkles. However, in a periphery of the bonded portion, that is, a non-bonded portion, nonwoven fabric 12 expands and wrinkle-shaped unevenness 91 is formed.

From a viewpoint of a degree of freedom in forming the unevenness 91, the micro-fibrous cellulose assembly 15 is preferably formed intermittently rather than being widely and continuously provided in the bonded region 82 of the liquid impervious resin film 11 and the nonwoven fabric 12. Since the unevenness 91 is formed on the bonded portion of the micro-fibrous cellulose assembly 15 and the periphery, when the micro-fibrous cellulose assembly 15 is widely and continuously provided, a ratio of a region around the bonded portion to the entire bonded region 82 in the nonwoven fabric 12 becomes relatively small. That is, a region in which the unevenness 91 is formed on the nonwoven fabric 12 is narrow. On the other hand, when the micro-fibrous cellulose assembly 15 is intermittently provided, the ratio becomes relatively large. That is, the region in which the unevenness 91 is formed becomes wide. When the unevenness 91 is formed in a wide range of the bonded region 82, the unevenness 91 having a gentle and natural texture is formed on the nonwoven fabric 12. However, in consideration of the design of the product, in some cases, the unevenness formed in the narrow region of the bonded region 82 may give a gentler and more natural impression.

The following can be exemplified as a mode of bonding the micro-fibrous cellulose assembly 15. In this mode, the bonded portion in which the liquid impervious resin film 11 and the nonwoven fabric 12 are bonded through the micro-fibrous cellulose assembly 15 and the non-bonded portion continuously or intermittently provided between bonded portions are alternately repeatedly provided, and the unevenness is formed on the nonwoven fabric 12 by the nonwoven fabric 12 expanding in the non-bonded portion and the nonwoven fabric 12 not expanding in the bonded portion. The micro-fibrous cellulose assembly 15 is not particularly limited. However, as illustrated in Fig. 3, in the bonded region 82 of the liquid impervious resin film 11 and the nonwoven fabric 12, the bonded portion may be formed over the entire surface or the non-bonded portion may be formed. To the non-bonded portion, for example, the micro-fibrous cellulose assembly 15 may not be applied, and adhesion by the hot melt adhesive 81 may not be provided. The hot melt adhesive 81 may be intermittently applied between the bonded portions of the micro-fibrous cellulose assembly 15 to form an adhesive portion (bonded portion). Then, the nonwoven fabric 12 does not expand in the bonded portion, and the nonwoven fabric 12 expands in the non-bonded portion.

In addition, the bonded portion and the non-bonded portion may be alternately repeatedly provided, or the bonded portion may be provided, for example, in a lattice or staggered shape, and the non-bonded portion may be provided in a region other than the bonded portion. However, the invention is not limited thereto.

When the micro-fibrous cellulose assembly 15 is attached to the nonwoven fabric 12 and cures (contracts), the unevenness is formed in the periphery of the bonded portion. By interposing the micro-fibrous cellulose assembly 15 between the liquid impervious resin film 11 and the nonwoven fabric 12, a part of the outer surface (nonwoven fabric 12) of the product becomes unevenness having gentile and natural texture. Thus, the appearance of the product is improved, and a soft impression is given, which is preferable. In addition, micro-fibrous cellulose has a large number of -OH groups on the surface and has high absorbability. Due to the high absorbability of the micro-fibrous cellulose assembly 15, the micro-fibrous cellulose assembly 15 retains moisture, and thus the outer surface and underwear are unlikely to feel damp.

Characteristically, the micro-fibrous cellulose assembly 15 is provided between the liquid impervious resin film 11 and the nonwoven fabric 12. As described above, the liquid impervious resin film 11 and the nonwoven fabric 12 may be bonded by welding, etc. When such a micro-fibrous cellulose assembly 15 is interposed between the liquid impervious resin film 11 and the nonwoven fabric 12, moisture permeating the liquid impervious resin film 11 is absorbed by the micro-fibrous cellulose assembly 15. When the sheet including the liquid impervious resin film 11 and the nonwoven fabric 12 having the bonded portion is used on the outer surface of the product, the moisture retains in the micro-fibrous cellulose assembly 15 rather than the nonwoven fabric 12 or clothing, and thus there is no risk of damaging the outer surface of the product or clothing.

In particular, it is preferable that the nonwoven fabric 12 is a water repellent nonwoven fabric since the micro-fibrous cellulose assembly 15 has higher absorbability. The water repellent nonwoven fabric can be produced by adding a water repellent agent to the nonwoven fabric (either internal addition or external addition may be used) by a known method. As the water repellent agent, it is possible to use silicone-based, paraffin-based, alkylchromic chloride-based water repellents, etc.

The micro-fibrous cellulose assembly 15 may be formed on the inner surface of the nonwoven fabric 12, or the sheet such as the nonwoven fabric or paper on which the micro-fibrous cellulose assembly 15 is formed may be placed between the liquid impervious resin film 11 and the nonwoven fabric 12. However, as illustrated in Fig. 10, it is preferable that the micro-fibrous cellulose assembly 15 is formed on the outer surface of the liquid impervious resin film 11. In particular, as the liquid impervious resin film 11 does not have absorbability, the nonwoven fabric 12 tends to have a moist feel. However, when the micro-fibrous cellulose assembly 15 is provided on the outer surface of the liquid impervious resin film 11, the nonwoven fabric 12 is unlikely to have a moist feel. In addition, when the micro-fibrous cellulose assembly 15 is formed on the liquid impervious resin film 11 made of a resin film having moisture permeability, the micro-fibrous cellulose assembly 15 has a shape of a film, and thus there is an advantage that the liquid impervious resin film 11 has improved water impermeability and strength and reduced elongation.

Preferred forms according to the invention will be exemplified below.

According to an embodiment of the invention,
it is possible to show a mode in which
the bonded portion with the micro-fibrous cellulose assembly 15 is provided in a striped shape, and
the liquid impervious resin film 11 and the nonwoven fabric 12 are attached through the hot melt adhesive 81 intermittently provided in a longitudinal direction of the bonded portion between bonded portions.

The bonded portion by the micro-fibrous cellulose assembly is provided in a striped shape. In the striped shape, for example, linear parts included in stripes may be arranged in the width direction WD, in the front-back direction LD, or obliquely at intervals. In addition, the linear parts may have a waveform, or the linear parts may be intermittent line segments.

In addition, the hot melt adhesive 81 can be applied by various methods such as bead, curtain, summit or spiral application, or pattern coating (transfer of hot melt adhesive by letterpress method). In particular, when the hot melt adhesive 81 is continuously applied in a spiral shape, the hot melt adhesive 81 is not applied to a range 83 surrounded by a spiral curve (see Fig. 20). Thus, a case where the hot melt adhesive 81 is continuously applied in the spiral shape can be regarded as intermittent application.

According to another embodiment,
it is possible to show a mode including
the absorber 56, and
the sheet member arranged on the back surface side of the absorber 56 so that the liquid impervious resin film 11 is on the absorber 56 side,
in which at least a part of the outer surface of the product is formed by the nonwoven fabric 12.

After preparing the absorber 56 and the sheet member including the liquid impervious resin film 11 and the nonwoven fabric 12 on the back surface side of the absorber 56, and attaching the absorber 56 and the sheet member by arbitrary attaching means (for example, attaching means using only the micro-fibrous cellulose assembly 15 or attaching means combining the micro-fibrous cellulose assembly 15 and the hot melt adhesive 81), the absorbent article can be manufactured through known processes. In particular, the liquid impervious resin film 11 side of the sheet member is directed to the absorber 56 side. In this way, it is possible to manufacture the absorbent article in which the wrinkle-shaped unevenness having the gentle and natural texture is formed on the nonwoven fabric 12 included in the outer surface of the product.

Hereinafter, specific patterns for bonding the hot melt adhesive 81 and the micro-fibrous cellulose assembly 15 in the bonded region 82 will be illustrated with reference to the drawings.

### [Pattern 1]

As illustrated in Fig. 9, pattern 1 is a mode in which the bonded portion of the micro-fibrous cellulose assembly 15 is provided in the striped shape in the bonded region 82 of the liquid impervious resin film 11 and the nonwoven fabric 12, and the hot melt adhesive 81 is applied to the entire region of the bonded region 82 to bond the liquid impervious resin film 11 and the nonwoven fabric 12.

In such a mode, the bonded portion of the micro-fibrous cellulose assembly 15 contracts and becomes hard by drying in the bonded region 82, and the unevenness 91 is formed at a position to which the hot melt adhesive 81 is not applied in the periphery of the bonded portion.

More specifically, for example, a plurality of attachment portions of the micro-fibrous cellulose assembly 15 is linearly arranged in parallel on the liquid impervious resin film 11 at intervals. Then, the hot melt adhesive 81 is applied from above to bond the liquid impervious resin film 11 and the nonwoven fabric 12. Here, an application method of the hot melt adhesive 81 is spiral coating, and in this spiral coating, there is a portion where the adhesive 81 is not applied, that is, a non-bonded portion in the range 83 surrounded by the spiral curve. In other words, the unevenness 91 is formed at a position to which the micro-fibrous cellulose assembly 15 is not attached and the hot melt adhesive 81 is not applied (see Figs. 10 and 12). In the nonwoven fabric 12 bonded to the liquid impervious resin film 11, the unevenness 91 is formed at a part facing the same position.

In this case, the liquid impervious resin film 11 and the nonwoven fabric 12 contract in the front-back direction LD and the width direction WD, and contract more in the width direction WD. A degree of contraction when a plurality of stripes of the micro-fibrous cellulose assembly 15 is arranged in the width direction WD is changed by the amount of attachment of the micro-fibrous cellulose assembly 15, a thickness of each strip, an interval between stripes before contraction, the amount of application of the hot melt adhesive 81, etc. For example, the degree of contraction can be represented by a contraction rate ((length WD of the member when the bonded portion of the micro-fibrous cellulose assembly 15 is provided)/(length WD of the member when the bonded portion of the micro-fibrous cellulose assembly 15 is not provided)). When the thickness of each stripe was 5 mm and the interval between stripes before contraction was 5 mm, the contraction rate was as follows.
(1) When the attachment amount of the micro-fibrous cellulose assembly 15 was 0.5 g/m², and the application amount of the hot melt adhesive 81 was 22 g/m², the contraction rate was 0.82.
(2) When the attachment amount of the micro-fibrous cellulose assembly 15 was 1.0 g/m², and the application amount of the hot melt adhesive 81 was 11 g/m², the contraction rate was 0.82.
(3) When the attachment amount of the micro-fibrous cellulose assembly 15 was 1.5 g/m², and the application amount of the hot melt adhesive 81 was 7 g/m², the contraction rate was 0.80.

Note that the contraction rate is affected by manufacturing error and manufacturing environment in a manufacturing process of the product.

A position where at least one of attachment of the micro-fibrous cellulose assembly 15 and application of the hot melt adhesive 81 to nonwoven fabric 12 is performed is the bonded portion, and a position where neither attachment of the micro-fibrous cellulose assembly 15 nor application of the hot melt adhesive 81 is performed is the non-bonded portion. As a result of not expanding the bonded portion of the nonwoven fabric 12 and expanding the non-bonded portion, the wrinkle-shaped unevenness 91 having a gentle and natural texture is formed on the nonwoven fabric 12.

In Fig. 9, on both side edges in the width direction WD, the bonded portions of the micro-fibrous cellulose assembly 15 are not provided, and the hot melt adhesive 81 is applied. However, it is possible to adopt the mode in which the micro-fibrous cellulose assembly 15 is arranged on the both side edges.

Note that in Fig. 9, the micro-fibrous cellulose assembly 15 is arranged in vertical stripes in plan view. However, the direction of the stripes is not limited to the vertical direction, and may be horizontal or oblique.

Conventionally, to form the unevenness on the product member, for example, as disclosed in Patent Literature 2, the outer body is provided with a combination of two types of elastic members having different elasticity, and the unevenness is generated due to the difference in elasticity. However, in this technology of Patent Literature 2, in order to combine two types of elastic members having different elasticity to prepare the outer body, expenditure is required for capital investment, etc. by installing separate equipment or making major design changes. In this respect, in the embodiment according to the invention, the unevenness can be formed by providing the bonded portion of the micro-fibrous cellulose assembly 15 on the product member. Thus, there is no need to spend for capital investment, etc., and the wrinkle-shaped unevenness having the gentle and natural texture can be formed on the nonwoven fabric 12 included in the outer surface of the product by a simple method, which is excellent in financial convenience.

### [Pattern 2]

As illustrated in Fig. 17, pattern 2 is a mode in which the bonded portion of the micro-fibrous cellulose assembly 15 is provided in the striped shape in the bonded region 82 of the liquid impervious resin film 11 and the nonwoven fabric 12, and the hot melt adhesive 81 is applied to a region in which the bonded portion of the micro-fibrous cellulose assembly 15 is not provided to bond the liquid impervious resin film 11 and the nonwoven fabric 12. Since the hot melt adhesive 81 is not applied to the entire bonded region 82, the amount of the hot melt adhesive 81 used can be reduced.

Even though the hot melt adhesive 81 is not applied to the bonded portion of the micro-fibrous cellulose assembly 15, the micro-fibrous cellulose assembly 15 has adhesiveness. Due to this adhesiveness, the liquid impervious resin film 11 and the nonwoven fabric 12 are bonded to each other through the micro-fibrous cellulose assembly 15 and do not move freely.

### [Pattern 3]

Pattern 3 is a mode in which the application amount of the hot melt adhesive 81 is reduced as illustrated in Fig. 13 as compared with pattern 1 and pattern 2. The bonded portions of the micro-fibrous cellulose assembly 15 are provided in the front-back direction LD along the hot melt adhesive 81 on both sides of the bonded portion of the hot melt adhesive 81, which extends in the front-back direction LD, in the width direction. The hot melt adhesive 81 and the bonded portion of the micro-fibrous cellulose assembly 15 are collectively set to a bonded portion S1, and a second bonded portion S2 is provided at an interval from the bonded portion S1 at one side in the width direction WD (right side in plan view), that is, by providing the non-bonded portion. Then, a third bonded portion S3 is provided at an interval from the bonded portion S2 at one side in the width direction (right side in plan view), that is, by providing the non-bonded portion. Similarly, a fourth bonded portion, a fifth bonded portion... may be repeated. Between adjacent bonded portions, the micro-fibrous cellulose assembly 15 is not attached, and the hot melt adhesive 81 is not applied. By using such a pattern, the amount of the hot melt adhesive 81 used can be reduced. In addition, unevenness is formed between the adjacent adhesive parts.

### [Pattern 4]

Pattern 4 is a mode obtained by modifying pattern 2. As illustrated in Fig. 14, this mode is a mode in which the bonded portion of the micro-fibrous cellulose assembly 15 is provided in the striped shape in the bonded region 82 of the liquid impervious resin film 11 and the nonwoven fabric 12, and the hot melt adhesive 81 is applied to a region in which the bonded portion of the micro-fibrous cellulose assembly 15 is not provided to bond the liquid impervious resin film 11 and the nonwoven fabric 12. In this mode, in a region in which the bonded portion of the micro-fibrous cellulose assembly 15 is not provided, a large amount-application portion (bonded portion) 81a to which a relatively large amount of the hot melt adhesive 81 is applied and a small amount-application portion (bonded portion) 81b to which a relatively small amount of the hot melt adhesive 81 is applied are alternately provided in the width direction. Describing in the figure, the large amount-application portion (bonded portion) 81a is provided along the front-back direction LD at a right end in the plan view, the bonded portion of the micro-fibrous cellulose assembly 15 is provided on the left side thereof in the front-back direction LD, the small amount-application portion (bonded portion) 81b is provided on the left side thereof in the front-back direction LD, the bonded portion of the micro-fibrous cellulose assembly 15 is provided on the left side thereof in the front-back direction LD, the large amount application portion (bonded portion) 81a is provided again on the left side thereof in the front-back direction LD, and this cycle is repeated. In the small amount application portion (bonded portion) 81b, the attachment amount of the hot melt adhesive 81 is smaller than that of the large amount application portion (bonded portion) 81a.

In addition, the following effects are obtained. When the micro-fibrous cellulose assembly 15 is attached to an attachment target, the wrinkle-shaped unevenness is formed around the bonded portion. The shape of the unevenness (wave) depends to some extent on the application amount of the hot melt adhesive 81 applied to the periphery of the bonded portion. That is, the shape of the unevenness (wave) changes depending on the application amount of the hot melt adhesive 81. Specifically, when the application amount is relatively large, the unevenness (wave) 91b having a relatively small amplitude and a short wavelength is formed. On the other hand, the inventors have found that, when the application amount is small, the unevenness (wave) 91a having a relatively large amplitude and a long wavelength is formed. When it is desired to form the unevenness (wave) having a relatively small amplitude and a short wavelength, the application amount of the hot melt adhesive 81 may be set to 11 to 30 g/m². When it is desired to form the unevenness (wave) having a large small amplitude and a long wavelength, the application amount of the hot melt adhesive 81 may be set to 2 to 10 g/m².

### [Pattern 5]

Pattern 5 is a mode obtained by modifying pattern 2. As illustrated in Fig. 9, this mode is a mode in which the bonded portion of the micro-fibrous cellulose assembly 15 is provided in the striped shape having a continuous linear part in the bonded region 82 of the liquid impervious resin film 11 and the nonwoven fabric 12, and the hot melt adhesive 81 is applied to a region in which the bonded portion of the micro-fibrous cellulose assembly 15 is not provided (between linear parts) to bond the liquid impervious resin film 11 and the nonwoven fabric 12. However, a difference from pattern 2 is that the bonded portions of the micro-fibrous cellulose assembly 15 are provided in the front-back direction LD intermittently with gaps 15c in the front-back direction LD, and the hot melt adhesive 81 is applied on both sides of the bonded portion in the width direction WD, so that the hot melt adhesive 81 is applied at an interval in the front-back direction LD. In this mode, since the bonded portions of the micro-fibrous cellulose assembly 15 are intermittently provided with the gaps 15c, the unevenness 91 is formed in the gaps 15c. A mode in which the unevenness 91 is formed in the gaps 15c is not each mode of pattern 1 to pattern 4, and gives an impression of the unevenness 91 having a natural texture different from pattern 1 to pattern 4.

### [Pattern 6]

As illustrated in Fig. 8, pattern 6 is a mode in which a plurality of bonded portions of the micro-fibrous cellulose assembly 15 is arranged in a dot shape with intervals in the bonded region 82.

Although not particularly limited, it is preferable that a size of each dot is 2 to 5 mm and an interval between dots is 5 to 10 mm.

Arrangement of the dot-shaped bonded portions of the micro-fibrous cellulose assembly 15 is not particularly limited. As an example, it is possible to present an arrangement that forms an orthorhombic lattice ((a) and (b) of the same figure), an arrangement that forms a lattice ((c) and (d) of the same figure), etc.

When the dot-shaped bonded portions are provided, as illustrated in Fig. 19, convexes 91t radially spread around the dot-shaped bonded portions, and the unevenness 91 (a region surrounded by a chain double-dashed line in the figure) is formed. Since a plurality of number of dot-shaped bonded portions is arranged, a plurality of pieces of unevenness 91 is provided. The unevenness 91 may have a shape similar to, for example, a flower shape or a star shape, which affects the appearance of the sheet member. Note that the unevenness 91 is formed to have a length 91L of 0 to 15 mm outward from the bonded portion.

### (Manufacturing Method)

An example of a method of manufacturing the sheet member having the liquid impervious resin film 11 and the nonwoven fabric 12 attached to one surface thereof will be described below.

The dispersion liquid of micro-fibrous cellulose is applied to at least one of facing surfaces of the liquid impervious resin film 11 and the nonwoven fabric 12 at intervals. In the case of application at intervals, the dispersion liquid can be applied in an arbitrary shape such as a shape of scattered dots, a lattice pattern, a striped shape, a wavy striped shape, or a staggered shape. In particular, the micro-fibrous cellulose dispersion liquid is more easily applied to the liquid impervious resin film 11. However, when it is desired to attach the micro-fibrous cellulose assembly to the inside of the fibers of the nonwoven fabric 12, the micro-fibrous cellulose dispersion liquid may be applied to the nonwoven fabric 12.

Next, the non-bonded portions which are continuously or intermittently provided between the bonded portions are alternately and repeatedly provided. To the non-bonded portion, for example, the micro-fibrous cellulose assembly 15 may not be applied, and adhesion by the hot melt adhesive 81 may not be provided. The hot melt adhesive 81 may be intermittently applied between the bonded portions of the micro-fibrous cellulose assembly 15 to form an adhesive portion (bonded portion). Then, the nonwoven fabric 12 does not expand in the bonded portion, and the nonwoven fabric 12 expands in the non-bonded portion. As an example, when the hot melt adhesive 81 is applied in the spiral shape (spiral application), the liquid impervious resin film 11 and the nonwoven fabric 12 are attached to each other in a spiral linear part, and are not attached to each other in a spiral gap part. In this gap part where attachment is not performed, the nonwoven fabric 12 is free (that is, the nonwoven fabric 12 and the liquid impervious resin film 11 are not bonded), and thus expansion occurs. In addition, in the linear part where attachment is performed, the nonwoven fabric 12 is not free (that is, the nonwoven fabric 12 and the liquid impervious resin film 11 are bonded), and thus expansion does not occur. This difference in expansion forms the unevenness 91.

Further, the liquid impervious resin film 11 and the nonwoven fabric 12 are bonded using a roller to form the bonded region 82 and dried. As a drying method, it is possible to adopt known methods such as natural drying and drying by blowing hot air (hot air drying). According to these methods, unevenness is formed at the bonded portion of the micro-fibrous cellulose assembly 15 formed on the target sheet and the periphery thereof. Further, hot air drying for rapid drying is suitable considering the production efficiency of the product. Furthermore, when the target sheet is dried at the glass transition temperature or higher, the unevenness 91 is formed not only on the bonded portion of the micro-fibrous cellulose assembly 15 and the periphery thereof on the target sheet but also on the target sheet. On the other hand, drying at a temperature lower than the glass transition temperature of the target sheet is preferable since the unevenness 91 is formed on the bonded portion and the periphery thereof.

### (Application of Sheet member to Absorbent article)

The absorbent article includes the absorber 56 and the sheet member formed of the liquid impervious resin film 11 and the nonwoven fabric 12 on the back surface side of the absorber 56, and can be manufactured by attaching the absorber 56 and the sheet member using arbitrary attaching means, and performing predetermined processes. In particular, the liquid impervious resin film 11 side of the sheet member is directed to the absorber 56 side. In this way, it is possible to manufacture the absorbent article in which the wrinkle-shaped unevenness having the gentle and natural texture is formed on the nonwoven fabric 12 included in the outer surface of the product.

The invention made by the inventors has been described above as an embodiment. However, the invention should not be limited by the description and drawings illustrating the present embodiment. Other examples, etc. performed by those skilled in the art based on the present embodiment are included in the invention.

### <Description of Terms Used Herein>

In a case where the following terms are used in the specification, those have the following meanings unless otherwise specified in the specification.
- The "front-back (vertical) direction" means a direction connecting the ventral side (front side) and the dorsal side (back side), and the "width direction" means a direction (right-left direction) orthogonal to the front-back direction.
- The "inner side" means a side close to the skin of the wearer, and the "outer side" means a side far from the skin of the wearer. The "inner surface" means a surface of a member close to the skin of the wearer, and the "outer surface" means a surface far from the skin of the wearer.
- "LD direction" and "WD direction" mean the flow direction (LD direction) in a manufacturing facility and the lateral direction (WD direction) orthogonal to the flow direction, and either one is the front-back direction of a product, and the other is the width direction of the product. The LD direction of a nonwoven fabric is the direction of fiber orientation of the nonwoven fabric. Fiber orientation is a direction along which a fiber of a nonwoven fabric runs and determined by, for example, a measurement method in accordance with the fiber orientation test method based on the zero span tensile strength of TAPPI standard method T481 and a simple measurement method for determining the direction of fiber orientation from the ratio of the tensile strength in the front-back direction to the width direction.
- "Spread state" means a flatly spread state without contraction or slack.
- "Stretch rate" means the value when the natural length is taken as 100%.
- "Gel strength" is measured as follows: 1.0 g of super absorbent polymer is added to 49.0 g of artificial urine (urea: 2 wt%, sodium chloride: 0.8 wt%, calcium chloride dihydrate: 0.03 wt%, magnesium sulfate heptahydrate: 0.08 wt%, and ion exchanged water: 97.09 wt%), and stirred with a stirrer. The resulting gel is left for three hours in a thermohygrostat bath at 40°C, 60%RH and then cooled to room temperature. The gel strength of the gel is measured with a curdmeter (Curdmeter-MAX ME-500, manufactured by I.techno Engineering).
- "Basis weight" is measured as follows. After the sample or test piece is preliminary dried, it is allowed to stand in a test room or apparatus under normal conditions (the test location is at a temperature: 23 ± 1°C, relative humidity: 50 ± 2%) to be constant weight. The preliminary drying is to make the sample or test piece be constant weight in an environment of a temperature of 100°C. Note that the fibers of an official moisture regain of 0.0% do not need preliminary drying. From a test piece having a constant weight, a sample having a size of 100 mm × 100 mm is cut out using a template for sampling (100 mm × 100 mm). The sample is weighed and the weight is multiplied by 100 into the weight per one square meter. The resulting value is defined as the basis weight.
- "Thickness" is automatically measured under the conditions of a load of 0.098 N/cm² in a pressurized area of 2 cm² using an automatic thickness measuring device (KES-G5 handy compression tester).
- "Water absorption capacity" is measured according to JIS K7223-1996 "Testing method for water absorption capacity of super absorbent polymers".
- "Water absorption speed" is the "time that elapses before the end point" measured in accordance with JIS K7224-1996 "Testing method for water absorption speed of super absorbent polymers" has been carried out using 2 g of superabsorbent polymer and 50 g of physiological saline solution.
- When environmental conditions in tests and measurements are not described, the tests and measurements shall be carried out in a test room or apparatus under normal conditions (the test location is at a temperature: 23 ± 1°C, relative humidity: 50 ± 2%).
- The dimension of each part means the dimension in the spread state, not the natural length state, unless otherwise stated.

### Industrial Applicability

The invention can be applied to general disposable diapers such as an underpants-type disposable diaper and a pad-type disposable diaper in addition to the tape-type disposable diaper as in the above example, and can be applied other absorbent articles such as sanitary napkins.

### Reference Signs List

- 11: LIQUID IMPERVIOUS RESIN FILM
- 12: NONWOVEN FABRIC
- 13: CONNECTING TAPE
- 13A: CONNECTING PORTION
- 13B: TAPE MAIN UNIT SECTION
- 13C: TAPE ATTACHING PORTION
- 15: MICRO-FIBROUS CELLULOSE ASSEMBLY
- 20: TARGET SHEET
- 21: END EDGE PORTION
- 30: TOP SHEET
- 40: INTERMEDIATE SHEET
- 50: ABSORBENT ELEMENT
- 56: ABSORBER
- 56W: ABSORBER WIDTH
- 58: WRAPPING SHEET
- 60: RISING GATHER
- 62: GATHER SHEET
- 81: HOT MELT ADHESIVE
- B: DORSAL SIDE PART
- F: VENTRAL SIDE PART
- WD: WIDTH DIRECTION
- LD: FRONT-BACK DIRECTION

## Claims

1. A sheet member comprising:
a liquid impervious resin film; and
a nonwoven fabric attached to one surface thereof,
wherein a bonded portion in which the liquid impervious resin film and the nonwoven fabric are bonded through a micro-fibrous cellulose assembly and a non-bonded portion continuously or intermittently provided between bonded portions are alternately repeatedly provided, and
unevenness is formed on the nonwoven fabric by the nonwoven fabric expanding in the non-bonded portion and the nonwoven fabric not expanding in the bonded portion.

2. The sheet member according to claim 1,
wherein the bonded portion with the micro-fibrous cellulose assembly is provided in a striped shape, and
the liquid impervious resin film and the nonwoven fabric are attached through a hot melt adhesive intermittently provided in a longitudinal direction of the bonded portion between the bonded portions.

3. The sheet member according to claim 1 or 2,
wherein the micro-fibrous cellulose assembly in the bonded portion includes 0.3 to 5.0 g/m² of micro-fibrous cellulose.

4. An absorbent article comprising:
an absorber; and
the sheet member recited in any one of claims 1 to 3 arranged on a back surface side of the absorber so that the liquid impervious resin film is on a side of the absorber,
wherein at least a part of an outer surface of a product is formed by the nonwoven fabric.

5. A method of manufacturing a sheet member including a liquid impervious resin film and a nonwoven fabric attached to one surface thereof, the method comprising:
applying a dispersion liquid of micro-fibrous cellulose at intervals to at least one of facing surfaces of the liquid impervious resin film and the nonwoven fabric; and then bonding and drying the liquid impervious resin film and the nonwoven fabric, thereby alternately repeatedly providing a bonded portion in which the liquid impervious resin film and the nonwoven fabric are bonded through a micro-fibrous cellulose assembly and a non-bonded portion continuously or intermittently provided between bonded portions.

6. A method of manufacturing an absorbent article including an absorber, and a liquid impervious resin film and a nonwoven fabric in this order on a back surface side of the absorber, at least a part of an outer surface of a product being formed by the nonwoven fabric, the method comprising
arranging a sheet member formed by the method recited in claim 5 so that the liquid impervious resin film is on a side of the absorber.
